# EUROPEAN PATENT SPECIFICATION

(11) **EP 2 468 849 B1**
(45) Date of publication and mention of the grant of the patent: **02.09.2015**
(21) Application number: 10809903.7
(22) Date of filing: 11.08.2010
(51) Int. Cl.: C12N 5/071, A61L 27/00, C12N 5/00, C12N 5/074

(54) **PROCESS FOR PRODUCTION OF BIOARTIFICIAL ORGAN**
VERFAHREN ZUR HERSTELLUNG BIOARTIFIZIELLER ORGANE
PROCÉDÉ DE PRODUCTION D ORGANE BIOARTIFICIEL

(30) Priority: 17.08.2009 JP 2009188578
(43) Date of publication of application: 27.06.2012
(73) Proprietor: Organ Technologies Inc., Minato-ku Tokyo 108-0074 (JP)
(72) Inventor: KITAMURA, Shinji, Okayama-shi Okayama 700-8558 (JP); MAKINO, Hirofumi, Okayama-shi Okayama 700-8558 (JP)
(74) Representative: Wasner, Marita
(86) International application number: PCT/JP2010/063659
(87) International publication number: WO 2011/021558

(56) References cited:
- WO-A1-2008/151254
- WO-A2-01/54706
- JP-A- 2004 520 295
- US-B1- 7 074 552
- KITAMURA SHINJI ET AL: "Establishment and characterization of renal progenitor like cells from S3 segment of nephron in rat adult kidney", FASEB JOURNAL, FED. OF AMERICAN SOC. FOR EXPERIMENTAL BIOLOGY, US, vol. 19, no. 13, 1 November 2005 (2005-11-01), pages 1789-1797, XP002471869, ISSN: 0892-6638, DOI: 10.1096/FJ.05-3942COM
- KINOMURA MASARU ET AL: "Amelioration of cisplatin-induced acute renal injury by renal progenitor-like cells derived from the adult rat kidney", CELL TRANSPLANTATION, vol. 17, no. 1-2, 2008, pages 143-158, XP009169305, ISSN: 0963-6897
- ROXANA PEY ET AL: "A new in vitro bioassay for cyst formation by renal cells from an autosomal dominant rat model of polycystic kidney disease", IN VITRO CELLULAR & DEVELOPMENTAL BIOLOGY - ANIMAL, vol. 35, no. 10, 1 November 1999 (1999-11-01), pages 571-579, XP055061740, ISSN: 1071-2690, DOI: 10.1007/s11626-999-0095-4
- DAVIES JA ET AL: "Genes and proteins in renal development", EXPERIMENTAL NEPHROLOGY, KARGER, DE, vol. 10, no. 2, 1 January 2002 (2002-01-01), pages 102-113, XP009088714, ISSN: 1018-7782
- KARIHALOO ANIL ET AL: "Signals which build a tubule", NEPHRON EXPERIMENTAL NEPHROLOGY, vol. 100, no. 1, 2005, pages E40-E45, XP002696448, ISSN: 1660-2129
- HIROYUKI TANAKA: 'Haisei Kansaibo (ES Saibo) kara no Bunka Yudo' JINZO vol. 28, no. 3, 2006, pages 175 - 179, XP003019964
- PEPICELLI C V ET AL.: 'GDNF induces branching and increased cell proliferation in the ureter of the mouse.' DEV BIOL vol. 192, no. 1, 1997, pages 193 - 198, XP008152382
- AKITO MAEJIMA: 'Basic Nephrology 7. Nyosaikan Saisei ni Kansuru Saikin no Chiken' ANNUAL REVIEW JINZO vol. 2009, January 2009, pages 43 - 49, XP008152394
- KIM D ET AL.: 'Nephrogenic factors promote differentiation of mouse embryonic stem cells into renal epithelia.' J AM SOC NEPHROL vol. 16, 2005, pages 3527 - 3534, XP002587898
- PERANTONI A 0 ET AL.: 'Basic fibroblast growth factor can mediate the early inductive events in renal development.' PROC NATL ACAD SCI USA vol. 92, no. 10, 1995, pages 4696 - 4700, XP008152387
- MARLIER A ET AL.: 'Genetic Diseases of the Kidney', February 2009 article 'An overview of renal development', pages 365 - 392, XP008152395
- SHINJI KITAMURA ET AL.: 'Seitai Jinzokan/Zenku Saibo kara no Jinzo Kozo Saikochiku - Baiyo Joken no Kento' THE JAPANESE JOURNAL OF NEPHROLOGY vol. 52, no. 3, May 2010, page 385, XP008152398
- MORIZANE R ET AL.: 'Differentiation of murine embryonic stem and induced pluripotent stem cells to renal lineage in vitro.' BIOCHEM BIOPHYS RES COMMUN vol. 390, no. 4, December 2009, pages 1334 - 1339, XP008152385
- SHINJI KITAMURA ET AL.: 'Seitai Jinzokan/Zenku Saibo kara no Jinzo Kozo Saikochiku to sono Kaiseki' REGENERATIVE MEDICINE vol. 9, February 2010, page 226, XP008155411
- SHINJI KITAMURA ET AL.: 'Seitai Jinzokan/Zenku Saibo kara no Jinzo Kozo Saikochiku to sono Kaiseki' THE JAPANESE JOURNAL OF NEPHROLOGY vol. 52, no. 3, May 2010, page 329, XP008152397
- Shinji Kitamura ET AL: "Single Adult Kidney Stem/Progenitor Cells Reconstitute Three-Dimensional Nephron Structures In Vitro", Stem Cells, vol. 33, no. 3, 17 March 2015 (2015-03-17) , pages 774-784, XP055179306, ISSN: 1066-5099, DOI: 10.1002/stem.1891

### SEQUENCE LISTING

<110> National University Corporation Okayama University
<120> A method for differentiating organ composed mature cells from immaturecells, reconstituting 3-dimentional organ structure, and medical reconstituting and regenerating organs for therapy
<130> OGTP1002F
<150> JP 2009-188578
   <151> 2009-8-17
<160> 22
<170> PatentIn version 3.1
<210> 1
   <211> 20
   <212> DNA
   <213> Artificial
<220>
   <223> Synthesized forward primer for detecting auaporin-1 gene
<400> 1
   cctccaggca cagtcttctc 20
<210> 2
   <211> 20
   <212> DNA
   <213> Artificial
<220>
   <223> Synthesized reverse primer for detecting auaporin-1 gene
<400> 2
   cagtggcctc ctgactcttc 20
<210> 3
   <211> 20
   <212> DNA
   <213> Artificial
<220>
   <223> Synthesized forward primer for detecting Na-Ca transporter gene
<400> 3
   tggaagctgg tctgtctcct 20
<210> 4
   <211> 25
   <212> DNA
   <213> Artificial
<220>
   <223> Synthesized reverse primer for detecting Na-Ca transporter gene
<400> 4
   tgatgacatc aagaaggtgg tgaag 25
<210> 5
   <211> 17
   <212> DNA
   <213> Artificial
<220>
   <223> Synthesized forward primer for detecting nephrin gene
<400> 5
   gctcccacca tccgtgc 17
<210> 6
   <211> 22
   <212> DNA
   <213> Artificial
<220>
   <223> Synthesized reverse primer for detecting nephrin gene
<400> 6
   gactatgtcc acacaacccc ca 22
<210> 7
   <211> 20
   <212> DNA
   <213> Artificial
<220>
   <223> Synthesized forward primer for detecting pax-2 gene
<400> 7
   agggcatctg cgataatgac 20
<210> 8
   <211> 20
   <212> DNA
   <213> Artificial
<220>
   <223> Synthesized reverse primer for detecting pax-2 gene
<400> 8
   ctcgcgtttc ctcttctcac 20
<210> 9
   <211> 20
   <212> DNA
   <213> Artificial
<220>
   <223> Synthesized forward primer for detecting gdnf gene
<400> 9
   cccgaagatt atcctgacca 20
<210> 10
   <211> 20
   <212> DNA
   <213> Artificial
<220>
   <223> Synthesized reverse primer for detecting gdnf gene
<400> 10
   tagcccaaac ccaagtcagt 20
<210> 11
   <211> 20
   <212> DNA
   <213> Artificial
<220>
   <223> Synthesized forward primer for detecting ret gene
<400> 11
   gcgtcaggga gatggtaaag 20
<210> 12
   <211> 20
   <212> DNA
   <213> Artificial
<220>
   <223> Synthesized reverse primer for detecting ret gene
<400> 12
   catcagggaa acagttgcag 20
<210> 13
   <211> 20
   <212> DNA
   <213> Artificial
<220>
   <223> Synthesized forward primer for detecting ncam gene
<400> 13
   acgtccggtt catagtcctg 20
<210> 14
   <211> 20
   <212> DNA
   <213> Artificial
<220>
   <223> Synthesized reverse primer for detecting ncam gene
<400> 14
   ctatgggttc cccatccttt 20
<210> 15
   <211> 20
   <212> DNA
   <213> Artificial
<220>
   <223> Synthesized forward primer for detecting Wt-1 gene
<400> 15
   acccaggctg caataagaga 20
<210> 16
   <211> 20
   <212> DNA
   <213> Artificial
<220>
   <223> Synthesized reverse primer for detecting Wt-1 gene
<400> 16
   gctgaagggc ttttcacttg 20
<210> 17
   <211> 20
   <212> DNA
   <213> Artificial
<220>
   <223> Synthesized forward primer for detecting AQP-2 gene
<400> 17
   gtggctgccc agctgctggg 20
<210> 18
   <211> 20
   <212> DNA
   <213> Artificial
<220>
   <223> Synthesized reverse primer for detecting AQP-2 gene
<400> 18
   agctccaccg actgccgccg 20
<210> 19
   <211> 20
   <212> DNA
   <213> Artificial
<220>
   <223> Synthesized forward primer for detecting bf-2 gene
<400> 19
   aggagacaga catcgacgtg 20
<210> 20
   <211> 19
   <212> DNA
   <213> Artificial
<220>
   <223> Synthesized reverse primer for detecting bf-2 gene
<400> 20
   tgacgaagca gtcgttgag 19
<210> 21
   <211> 25
   <212> DNA
   <213> Artificial
<220>
   <223> Synthesized forward primer for detecting gapdh gene
<400> 21
   tgatgacatc aagaaggtgg tgaag 25
<210> 22
   <211> 23
   <212> DNA
   <213> Artificial
<220>
   <223> Synthesized reverse primer for detecting gapdh gene
<400> 22
   tccttggagg ccatgtaggc cat 23

## Description

### TECHNICAL FIELD

The present invention relates to a method for inducing the differentiation of renal stem cells to tissue cells using cultured cells. The present invention also relates to a method for producing a bioartificial kidney comprising differentiation-induced tissue cells, and further relates to a material for medical purposes comprising the obtained bioartificial kidney.

### BACKGROUND ART

In recent years, tissue engineering has received attention where a function is recovered and an organ and tissue are regenerated in the organ and the tissue whose functions were lost or reduced due to disease or accident, etc. Culture conditions and factors for allowing stem cells and undifferentiated cells to proliferate and differentiate into objective cells have been studied in this tissue engineering field. Tissue engineering generally requires three elements, that is, a cell, a growth factor and an anchorage on which the cell can be engrafted. An attempt has been made that cells are cultured on the anchorage and the growth factor is bound to construct a regenerated tissue.

Chemically synthesized articles such as polymers are used as the anchorage of cell proliferation for morphologically reconstructing the organ and the tissue in tissue engineering, and are also referred to as a "scaffold." Methods have often been reported that objective cells are seeded on this scaffold and cultured under an appropriate environment in vitro to proliferate and differentiate them into required cells and that the cells are proliferated in a three dimensional structure to regenerate a desired organ by grafting the scaffold in a defect of an organ or tissue to be regenerated to proliferate and differentiate the objective cells into the required cells in vivo (Patent Literatures 1 and 2). However, it is very difficult to proliferate the cells in a three dimensional structure without using such an anchorage. Further, the chemically synthesized scaffold has a drawback that it cannot regenerate the organ and the tissue even though it can mimic the three dimensional construction intrinsic to living bodies. Under actual conditions, even though a bioartificial organ is constructed using the scaffold, the function of the organ cannot be elicited sufficiently and that it is difficult to construct the bioartificial organ itself regardless of the presence or absence of the scaffold.

An embryonic stem cell (hereinafter also simply referred to as an "ES cell") is an undifferentiated cell induced from an early embryo referred to as a blastocyst, and is a cell that has totipotency for self-replication and differentiation. The ES cell can differentiate into all types of cells including germ cells only by culturing as an assembly mass in vitro. When the ES cells are differentiated into functional cells required for regenerative medicine such as a cell therapy, first it is necessary to form an embryoid body having the three dimensional structure. Methods of culturing the ES cells for forming the embryoid body include a hanging drop method (Non-Patent Literature 1), a suspension culture, a culture in a semisolid medium containing methylcellulose, etc., and combinations thereof.

The number of patients with chronic renal failure has increased in recent years. In chronic renal failure, renal disorder caused by a renal disease or a disease in an organ other than a kidney progresses, functions that a normal kidney has, such as the function of removing urinary toxins contained in blood, an endocrine function and a regulatory function and the like are lost. When chronic renal failure progresses, functions of a normal kidney are lost, and thus each organ in the body is affected to cause uremia. Specifically, central nervous disorders, peripheral nervous disorders, cardiac/cardiovascular disorders, gastrointestinal disorders, visual and ocular disorders, blood coagulation disorders, immunological disorders, endocrine disorders, cutaneous disorders, bone and joint disorders, electrolyte disorders, acid-base balance disorders and the like are caused. Chronic renal failure is progressive and its cure is impossible. Therefore, treatment has focused on a conservative treatment that delays the speed of losing renal functions, and finally requires treatment such as artificial dialysis and kidney transplantation, which alternates renal functions (for example, Patent Literature 3).

However, it is difficult to perform kidney transplantation because the number of kidneys to be provided is absolutely insufficient. Artificial dialysis is broadly classified into hemodialysis and peritoneal dialysis. In hemodialysis, although urinary toxins in the blood are removed, treatment cannot be carried out to replace the endocrine function and the regulatory function, etc. The patient is required to undergo hemodialysis about three times a week, etc., which is a great burden on the patient. Peritoneal dialysis can be managed by the patient by himself, and thus the patient can reduce the frequency of visits to a clinic, but problems occur such as the patient easily undergoes a metabolic disorder and the burden on the peritoneum becomes great.

Concerning a kidney, it has been disclosed that renal stem cells/precursor cells capable of high proliferation were isolated from a rat kidney and the rKS (rat kidney stem cell line) 56 cell line established from these was acquired (Patent Literature 4) . In Patent Literature 4, it has been disclosed that the rKS56 cell line is capable of self-proliferation and when cultured in a system containing a culture medium with support cells (feeder cells), a vascular endothelial cell marker was detected to suggest the possibility of differentiating into vascular endothelial cells. It has also been disclosed that the rKS56 cells can be differentiated into a loop of Henle cells, proximal convoluted tubular cells and collecting duct (CD) cells. However, the method is not reported that the rKS56 cells were differentiated in the system not containing a support cell (feeder cells). It is also not reported that a bioartificial organ composed of a three dimensional structure was produced using the above differentiated cells.

### RELATED ART REFERENCES

### PATENT LITERATURE

Patent Literature 1: Japanese Published Unexamined Patent Application No. 2008-049146
Patent Literature 2: Japanese Published Unexamined Patent Application No. 2009-039401
Patent Literature 3 : Japanese Published Unexamined Patent Application No. 6-142193
Patent Literature 4 : Japanese Published Unexamined Patent Application No. 2004-275079

### Non-Patent Literature

Non-Patent Literature 1: J. Cell Biol., 126 (3): 701-711 (1994).

### SUMMARY OF INVENTION

### PROBLEM TO BE SOLVED BY THE INVENTION

It is an object of the present invention to provide a method for inducing the differentiation of renal stem cells into renal tissue cells and provide a bioartificial kidney comprising the obtained tissue cells. It is another object of the present invention to provide a material for medical purposes comprising the produced bioartificial kidney.

### MEANS FOR SOLVING PROBLEMS

As a result of extensive study for achieving the above objects, the present inventors have found that renal stem cells can be differentiated into renal tissue cells by culturing renal stem cells in a culture medium containing fetal calf serum, a hepatic cell growth factor, a glial cell line-derived neurotrophic factor, a basic fibroblast growth factor, a bone morphogenetic protein 7 and an epithelial cell growth factor in a base medium in the presence of a three dimensional scaffold, and completed the present invention.

That is, the present invention relates to the method for inducing the differentiation of renal stem cells into renal tissue cells, and the above method for inducing the differentiation is characterized in that renal stem cells are cultured in a three dimensional manner in the culture medium containing the hepatic cell growth factor (HGF), the glial cell line-derived neurotrophic factor (GDNF), the basic fibroblast growth factor (b-FGF), the bone morphogenetic protein 7 (BMP7) and the epithelial cell growth factor (EGF) in the base medium in the presence of the three dimensional scaffold.

Here, in one embodiment of the method for inducing the differentiation of the present invention, it is preferable that the three dimensional culture comprises a first step of culturing renal stem cells in the culture medium containing HGF, GDNF and b-FGF and a second step of culturing in a culture medium containing HGF, GDNF, b-FGF, BMP7 and EGF after 0 to 10 days have passed from the start of the culture in the first step.

In one embodiment of the method for inducing the differentiation of the present invention, it is preferable to culture under a condition where HGF, GDNF, b-FGF and BMP7 in a concentration each independently selected from 100 to 500 ng/mL and EGF in a concentration selected from 300 to 700 ng/mL are contained in the culture medium. Also, in one embodiment of the method for inducing the differentiation of the present invention, it is preferable to further contain the serum in the culture medium. In the method for inducing the differentiation of the present invention, the stem cell is preferably a renal stem cell.

One embodiment of the method for inducing the differentiation of the present invention is the method for inducing the differentiation comprising
1) a step of forming a three dimensional cell mass from the renal stem cells, and
2) a step of culturing the cell mass in a three dimensional manner in the culture medium containing HGF, GDNF, b-FGF, BMP7 and EGF in the presence of the three dimensional scaffold.

Here, the above step 2) can comprise the first step of culturing the cell mass in the three dimensional manner in the culture medium containing HGF, GDNF and b-FGF in the presence of the three dimensional scaffold (Step 2A) and the second step of culturing in the three dimensional manner in culture medium containing HGF, GDNF, b-FGF, BMP7 and EGF after 0 to 10 days have passed from the start of the culture in the first step (Step 2B).

In the above step 1), it is preferable that the cell mass is formed by the hanging drop method.

According to another embodiment of the present invention, the present invention relates to a tissue cell induced and formed by the above method for inducing the differentiation.

Here, the tissue cell induced and formed by the above method for inducing the differentiation of the present invention can be a renal tissue cell in one embodiment.

According to still another embodiment of the present invention, the present invention relates to a method for producing the bioartificial kidney, and the method is characterized by comprising
1) a step of forming a cell mass of renal stem cells,
2) a step of seeding the obtained cell mass on the scaffold, and
3) a step of culturing the cell mass in the culture medium containing HGF, GDNF, b-FGF, BMP7 and EGF on the scaffold.

Also according to still another embodiment of the present invention, the present invention relates to a method for inducing the differentiation of the renal stem cells into renal tissue cells having a polycystic kidney-like morphological feature, and the method for inducing the differentiation is characterized by comprising
1) a step of forming a three dimensional cell mass from the renal stem cells; and
2) a step of culturing the obtained three dimensional cell mass from the renal stem cells in a three dimensional manner in the culture medium containing BMP7 and EGF and not containing at least one of GDNF, b-FGF or HGF in the presence of the three dimensional scaffold.

In the method for inducing the differentiation into the renal tissue cells having the polycystic kidney-like morphological feature, it is preferable that the culture medium in the step 2) contains BMP7 and EGF and contains none of GDNF, b-FGF or HGF in one embodiment.

Also according to still another embodiment of the present invention, the present invention relates to the renal tissue cells which are induced and formed by the method for inducing the differentiation into the renal tissue cells having the polycystic kidney-like morphological feature and which have the polycystic kidney-like morphological feature.

### EFFECTS OF THE INVENTION

By the method for inducing the differentiation of the renal stem cells into the renal tissue cells of the present invention, it was possible to induce the differentiation of the undifferentiated cells into the tissue cells and further construct a three dimensional structure. Specifically, according to the methods of the present invention, it was possible to differentiate the renal stem cells/precursor cells into distal convoluted tubule cells, glomerular cells, proximal convoluted tubule cells, and the like of the kidney, and further it was possible to reconstruct a kidney-like structure in vitro.

### BRIEF DESCRIPTION OF DRAWINGS

FIG. 1 is a schematic view showing a structure of a kidney.
FIG. 2 is an outline view showing the method for inducing the differentiation of the present invention (Example 1).
FIG. 3 is a photographic view showing a cultured morphological feature on Day 21 of the three dimensional culture. (a) shows the morphological feature of the rKS cell mass on Day 21 of the three dimensional culture, (b) shows the morphological feature of a metanephric mesenchymal stem cell on Day 21 of the three dimensional culture, and (c) shows the morphological feature of ureteric bud cells on Day 21 of the three dimensional culture (Examples 1 and 2).
FIG. 4 is a photographic view showing cultured morphological features of rKS cells on Day 21 of the three dimensional culture. A shows a state where no lumen structure was observed, B shows a state where no assembled calices-like structure was observed and a glomerulus-like structure plus a urinary duct-like structure was observed, and C shows a state where all of the glomerulus-like structure, the urinary duct-like structure and the calices-like structure were observed (Example 3).
FIG. 5 is a view showing a ratio of each morphological feature, which changed depending on the number of cells used upon producing the rKS cell mass, on Day 21 of the three dimensional culture of the rKS cells. Descriptions of A, B and C are the same as described in FIG. 4 (Example 3).
FIG. 6 is a photographic view showing the cultured morphological features of the of rKS cell on Day 1, Day 7 and Day 21 of the three dimensional culture when the number of cells was changed upon producing the rKS cell mass (Example 3).
FIG. 7 is a photographic view showing the cultured morphological features of the rKS cell on Day 21 of the three dimensional culture in each culture condition. The cultured morphological feature on Day 4 of the three dimensional culture is shown in m (Example 4).
FIG. 8 is a photographic view showing the cultured morphological features of the rKS cell on Day 4 of the three dimensional culture (Example 5).
FIG. 9 is a photographic view showing surface markers on the cultured cells on Day 4 of the three dimensional culture of the rKS cells (Example 1).
FIG. 10 is a photographic view showing the morphological features of rKS cells after the three dimensional culture for two weeks (Example 6).
FIG. 11 is a photographic view showing the morphological features of rKS cells after the three dimensional culture for three weeks (Example 6).
FIG. 12 is a photographic view showing the morphological features of the of rKS cells after the three dimensional culture for three weeks (Example 6).
FIG. 13 is micrographs showing the morphological feature of a distal convoluted tubule after the three dimensional culture of the rKS cells for three weeks. The arrow in the figure indicates primary cilla (Example 6).
FIG. 14 is micrographs showing the morphological feature of the glomerulus after the three dimensional culture of the rKS cells for three weeks (Example 2).
FIG. 15 is micrographs showing the morphological feature of a proximal convoluted tubule after the three dimensional culture of the rKS cells for two weeks (Example 6).

### MODES FOR CARRYING OUT THE INVENTION

The present invention relates to the method for inducing the differentiation of the stem cells into organ cells. In the present invention, the stem cell refers to a cell capable of differentiating into a specific cell when directed to change into a specific cell. The stem cell is classified into an embryonic stem cell (ES cell), an adult stem cell, an embryonic germcell, and the like, and the stem cell of the present invention may be any of these cells. Considering the ease in handling, it is suitable to use the adult stem cell or the embryonic germ cell.

The adult stem cell refers to a cell collected from the tissue in a living body and before being differentiated. Undifferentiated cells, i.e., stem cells as well as already differentiated mature cells are included in the tissue. The adult stem cell can produce any individual cells to be present by being differentiated in the tissue as well as being capable of replicating and producing the same cell as itself. The stem cell or the precursor cell isolated from the tissue in the living body by a conventionally well-known method can be used as the stem cell that can be used in the present invention. Specifically, for example, the stem cell of the present invention includes the renal stem cell/precursor cell described in Patent Literature 4. The description of the "stem cell" herein and claims in the present application is a concept construed in a broad sense and including not only the stem cells but also the precursor cells. The description of the "stem cell/precursor cell" is appropriately used for meaning "stem cell" in the broad sense.

The culture medium for a three dimensional culture used for the method for inducing differentiation of stem cells into tissue cells of the present invention is characterized by comprising fetal calf serum (hereinafter, "FCS"), the hepatic cell growth factor (hereinafter, "HGF"), the glial cell line-derived neurotrophic factor (hereinafter, "GDNF"), the basic fibroblast growth factor (hereinafter, "b-FGF"), the bone morphogenetic protein 7 (hereinafter, "BMP7") and the epithelial cell growth factor (hereinafter, "EGF") in the base medium. A concentration of FCS contained in the culture medium can be about 10%v/v. HGF can be contained in a concentration selected from 10 to 500 ng/mL and preferably 200 to 400 ng/mL, and more preferably in a concentration of 250 ng/mL. GDNF can be contained in a concentration selected from 100 to 500 ng/mL and preferably 200 to 400 ng/mL, and more preferably in a concentration of 250 ng/mL. Also, b-FGF can be contained in a concentration selected from 100 to 500 ng/mL and preferably 200 to 400 ng/mL, and more preferably in a concentration of 250 ng/mL. BMP7 can be contained in a concentration selected from 100 to 500 ng/mL and preferably 200 to 400 ng/mL, and more preferably in a concentration of 250 ng/mL. EGF canbe contained in a concentration selected from 300 to 700 ng/mL and preferably 400 to 600 ng/mL, and more preferably in a concentration of 500 ng/mL.

As described above, in the embodiments, in the method for inducing the differentiation of the present invention comprising
1) a step of forming the three dimensional cell mass from the stem cells; and
2) a step of culturing the cell mass in the three dimensional manner in the culture medium containing HGF, GDNF, b-FGF, BMP7 and EGF in the presence of the three dimensional scaffold, when the above step 2) comprises the first step (step 2A) of culturing the cell mass in the three dimensional manner in the culture medium containing HGF, GDNF and b-FGF in the presence of a three dimensional scaffold and the second step (step 2B) of culturing the cell mass in the three dimensional manner in the culture medium containing HGF, GDNF, b-FGF, BMP7 and EGF after 0 to 10 days have passed from the start of the culture in the first step, for example, the preferable concentrations of each component in the culture medium in the first and second steps can be set up as follows. That is, the culture medium in the first step is prepared by combining FCS, HGF, GDNF and b-FGF so that the above preferable concentrations are realized. The culture medium in the second step can also be prepared by adding BMP7 and EGF into the culture medium in the first step so that the preferable final concentrations are realized.

Here, it is preferable to use the DMEM/F12 medium as the base medium for the culture medium. However, the base medium is not limited thereto, and a base medium well-known in the art can also be used. Further, antibiotics such as penicillin and streptomycin can appropriately be added to the culture medium as needed.

The induction of the differentiation of the stem cells into the tissue cells of the present invention can be achieved by the three dimensional culture using the above culture medium in the presence of the three dimensional scaffold. When the three dimensional culture is performed, it is suitable to first comprise the step of forming the three dimensional cell mass from the stem cells.

More specifically, the following steps are included:
1) a step of forming the three dimensional cell mass from the stem cells; and
2) a step of culturing the obtained cell mass in the three dimensional manner in the culture medium containing FCS, HGF, GDNF, b-FGF, BMP7 and EGF in the presence of the three dimensional scaffold.

In the above, the method for producing the three dimensional cell mass is not particularly limited, and for example, the three dimensional cell mass can be produced by the hanging drop method. The hanging drop method includes a method, for example, in which a cell suspension containing the stem cells of the present invention prepared by using the solution shown below for producing the three dimensional cell mass is placed on a lid, etc., of a culture plate and cultured upside down so that the placed cell suspension is hung down. Specifically, it is possible to prepare the cell suspension containing 1×10⁴ to 1×10⁶ cells, preferably 5×10⁴ to 5×10⁵ cells and more preferably 1×10⁵ to 2×10⁵ cells in 50 uL of the solution for producing the cell mass. A culture time for forming the cell mass is suitably about 6 to 10 hours. When the culture time is shorter than 6 hours, a sufficient cell mass cannot be obtained. When it is longer than 10 hours, the formed cell mass breaks up. Thus, it is preferable to culture for 6 to 10 hours at 37°C. The cell mass produced by the hanging drop method can become an embryoid body when the stem cell is an ES cell. Here, the embryoid body refers to an embryo formed to induce the differentiation of the ES cells into various tissues. The formation of the embryoid body is a first step of inducing the differentiation of the ES cells in vitro. It is considered that the formation of the three dimensional cell mass allows intercellular signals for cell differentiation to act to differentiate the stem cells more easily.

When the three dimensional cell mass is produced in the above, for example, it is preferable to use a solution containing FCS, insulin, transferrin, selenium, dexamethasone, HGF and b-FGF in the base medium. Here, it is preferable to use a DMEM/F12 medium as the base medium. However, the base medium is not limited thereto, and a base medium well-known in the art can also be used. A commercially available ITS-mix (manufactured by Gibco) can also be used as the solution containing insulin, transferrin and selenium. The concentration of serum (for example, FCS) contained in the solution can be about 10%v/v. Also, insulin can be contained in a concentration of 1 to 10 g/mL and more preferably about 5 g/mL. Also, transferrin can be contained in a concentration of 1 to 5 g/ml and more preferably about 2.75 g/mL. Also, selenium (sodium selenite) can be contained in a concentration of 1 to 5 ng/mL and more preferably about 3.35 ng/mL. Also, dexamethasone can be contained in a concentration of 1 to 10×10⁻⁸ M and more preferably 5×10⁻⁸ M. Also, HGF can be contained in a concentration of 1 to 100 ng/mL and more preferably 5 ng/mL. Also, b-FGF can be contained in a concentration of 1 to 100 ng/mL and more preferably 25 ng/mL. Antibiotics such as penicillin and streptomycin can also appropriately be added to this solution as needed.

In the method for inducing the differentiation of the stem cells into the tissue cells of the present invention, the stem cells can be cultured in the above culture medium for the three dimensional culture in the presence of the three dimensional scaffold. More preferably, the three dimensional cell mass formed from the above stem cells can be cultured using the aforementioned culture medium for the three dimensional culture.

The three dimensional culture can be performed in the above-described culture medium of stem cells, i.e., the culture medium containing FCS, HGF, GDNF and b-FGF, as well as BMP7 and EGF, or can be performed by dividing the three dimensional culture into two stages, i.e., culturing in advance in the culture medium containing FCS, HGF, GDNF and b-FGF in the above concentrations and further culturing under the conditions containing BMP7 and EGF in the above concentrations in addition to the culture medium containing FCS, HGF, GDNF and b-FGF after 0 to 10 days have passed. When the three dimensional culture is divided into the two stages, the second step (step 2B) of culturing in the culture medium containing FCS, HGF, GDNF, b-FGF, BMP7 and EGF can be performed after preferably 1 to 8 days and more preferably 4 to 7 days have passed from the start of the first step (step 2A) of culturing in the culture medium containing FCS, HGF, GDNF and b-FGF. A rate of inducing the differentiation can be improved by changing from the culture medium in the first step to the culture medium in the second step in the above preferable period. The concentrations of BMP7 and EGF to be added can also be gradually increased in a gradient manner so as to finally reach the concentrations shown above. For example, the culture medium containing HGF, GDNF and b-FGF is added in a lower section of a double chamber for culture, the culture medium containing BMP7 and EGF is added in an upper section thereof, and includes methods such as a concentration gradient can be made by gradually transferring the culture medium containing BMP7 and EGF in the upper section to the lower section with culturing the aforementioned stem cells in the culture medium in the lower section.

As the serum contained in the culture medium used for the method for inducing the differentiation of the stem cells into the tissue cells, FCS is mainly described herein, but the serum used in the base medium is not limited to FCS, and it is possible to use sera, such as other bovine serum, human serum, and the like, which are capable of serving the desired roles and well-known in the art.

A material for the three dimensional scaffold used in the present invention is not particularly limited as long as they are publicly known in the art, and for example, collagen-based materials, polymer-based materials such as polycaprolactone and polyglycolic acid, or composites thereof can be used. Its morphological feature is also not particularly limited, and includes, for example, a sponge-shaped structure, etc. The three dimensional scaffold may be those using samples derived from the living body (for example, extracellular matrix and basal membrane, etc.) as the materials. Specifically, Matrigel^{™} (Becton, Dickinson and Company), type I collagen gel and type IV collagen gel and the like can be included.

The present invention also extends to the tissue cells obtained by the method for inducing the differentiation of the stem cells into the tissue cells, and also extends to the bioartificial organ formed by the tissue cells. For example, when the stem cell used is the renal stem cell/precursor cell, the obtained tissue cell is a renal cell, and the bioartificial organ formed from the tissue cells is a bioartificial kidney. The method for acquiring the renal stem cell/precursor cell is not particularly limited, and the renal stem cell/precursor cell can be acquired according to the method described in Patent Literature 4, for example. The present invention also extends to the material for medical purposes comprising the bioartificial organ produced in this way.

### EXAMPLES

Hereinafter, the present invention is specifically described with reference to Examples for promoting better understanding, but it is a matter of course that these do not limit the scope of the present invention.

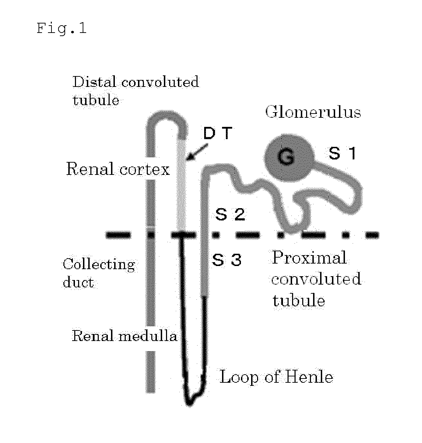

Two kidneys are present in a dorsal side of a peritoneal cavity in a human, each of which has a urinary duct extending to a urinarybladder, producing and secreting hormones and producing urine, and mainly having an excretion function, an endocrine function and a regulatory function. Approximately a million of the basic structures referred to as nephron are present in the kidney, and comprise the glomerulus that filtrates water including waste from blood to produce primitive urine. The primitive urine produced in the glomerulus is carried to a urinary duct through a proximal convoluted tubule, a loop of Henle, a distal convoluted tubule and a collecting tubule. Sodium and water, etc., are reabsorbed and erythropoietin and vitamin D3 are activated in the proximal convoluted tubule (S1: segment 1, S2: segment 2 and S3: segment 3), the loop of Henle and the distal convoluted tubule. The nephron is surrounded with interstitial cells, etc. The primitive urine is concentrated to about 100 times until being carried to the urinary duct, then carried to the urinary bladder through the urinary duct, and excreted from the urinary bladder out of the body (see FIG. 1).

The kidney is developed from intermediate mesoderm, and formed through three stages, i.e., pronephros, mesonephros and metanephros. In mammalian animals, the pronephros and the mesonephros are subsequently nearly degenerated, and the kidney is mainly formed from the metanephros. Mesenchymal cells assemble aroundprocesses referred to as uretericbuds developed on the most tail side of the mesonephros to form the metanephros. The ureteric buds are interacted with the mesenchymal cells and the mesenchymal cells are epithelized to form a sigmoid body, thereby developing the glomerulus, the proximal convoluted tubule, the loop of Henle and the distal convoluted tubule. The collecting tubule and urinary duct epithelial cells and the like are differentiated from the ureteric buds.

### (Example 1) Three dimensional culture using renal stem cells/precursor cells

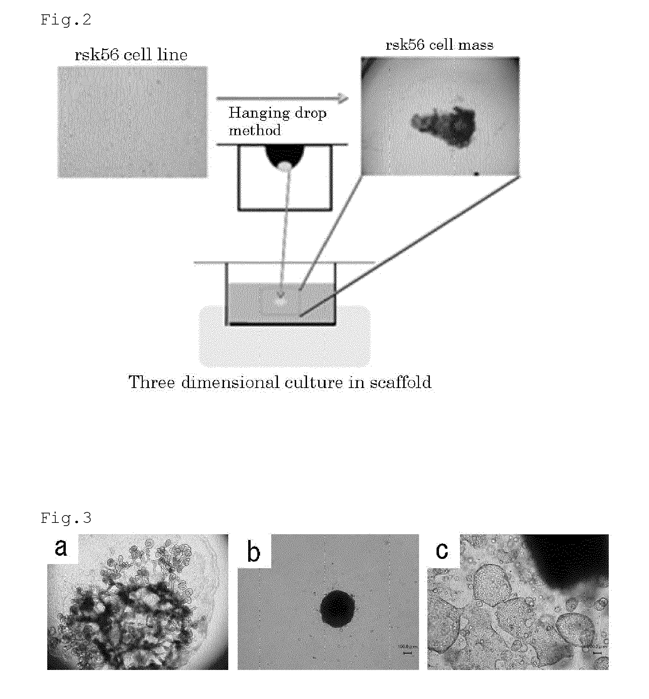

The three dimensional culture was carried out by culturing renal stem cells/precursor cells by a hanging drop method and culturing the obtained cell mass in a scaffold composed of Matrigel^{™} (see FIG. 2).

### 1) Renal stem cells/precursor cells

Cells were isolated from the S3 region of the proximal convoluted tubule according to the method described in Patent Literature 4, and an SV40 gene was incorporated into the obtained cells capable of high proliferation by a lipofection method to establish a cell line. The obtained cell line is composed of the renal stem cells/precursor cells of the present Example and is hereinafter referred to as an rKS56 cell.

### 2) Production of cell mass of rKS56 cells

A solution containing FCS (final concentration: 10%v/v), ITS-mix (manufactured by Gibco) (200 µL), dexamethasone (final concentration: 5×10⁻⁸ M), HGF (final concentration: 5 ng/mL), and b-FGF (final concentration: 25 ng/mL) in DMEM/F12 medium was prepared. A cell suspension containing about 1×10⁵ rKS56 cells in 50 µL of the above solution was prepared, placed on an inner surface of a lid of a 96-well microplate, and the lid was taken back on the microplate to perform the hanging drop culture. The cells were cultured at 37°C for 6 to 10 hours to produce a cell mass of the rKS56 cells (see FIG. 3).

### 3) Three dimensional culture of rKS56 cells

A culture medium containing FCS (final concentration: 10%v/v), HGF (final concentration: 250 ng/mL), GDNF ((final concentration: 250 ng/mL) and b-FGF (final concentration: 250 ng/mL) in a solution obtained by mixing a DMEM/F12 medium and Matrigel^{™} (Becton, Dickinson and Company) at 1: 1 was prepared. First, as the first step (Step 2A) of the three dimensional culture, one cell mass produced in 2) above was seeded in one well of a 24-well Transwell (Corning, Cat# 3470) using the above culture medium containing Matrigel^{™} as the scaffold, and cultured at 37°C for 5 to 7 days. Subsequently, as the second step (step 2B) of the three dimensional culture, the cell mass was cultured at 37°C for about 4 weeks in the culture medium additionally containing BMP7 (final concentration: 250 ng/mL) and EGF (final concentration: 500 ng/mL) in the culture medium containing FCS, HGF, GDNF andb-FGF. As one Example, the culture medium in the first step (step 2A) was changed to the culture medium in the second step (step 2B) by adding 2 µL of a BMP7 high-concentration solution at 50 µg/mL and 2 µL of an EGF high-concentration solution at 100 µg/mL in each well of the 24-well Transwell containing 400 µL of the culture medium containing FCS, HGF, GDNF andb-FGF. A cultured morphological feature on Day 21 of the three dimensional culture is shown in FIG. 3a.

### (Example 2) Three dimensional culture using renal stem cells/precursor cells

Metanephric mesenchymal stem cells or ureteric bud cells were used in place of the rKS56 cells, and cultured in the same manner as in Example 1. The cultured morphological features on Day 21 of the three dimensional culture are shown in FIGs. 3b and 3c, respectively.

### (Example 3) Morphological features of cultured cells depending on cell number used for producing a cell mass of rKS56 cells

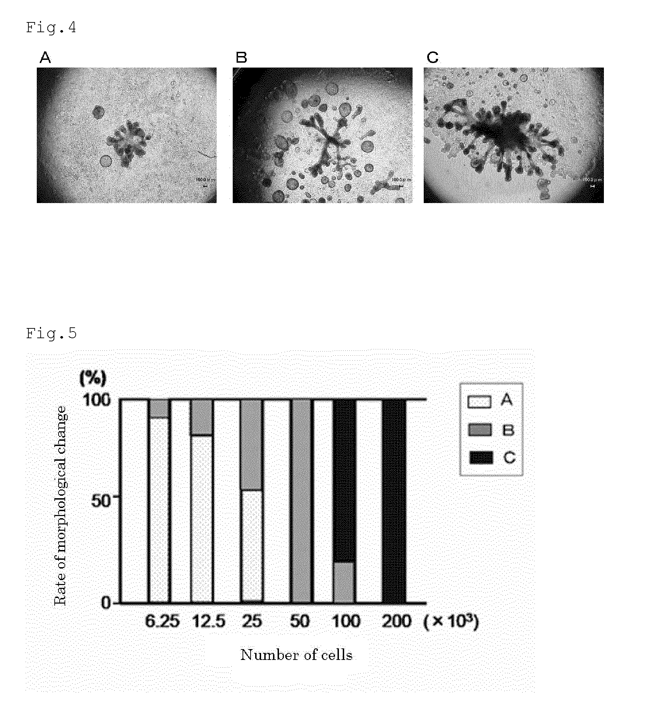

The rKS56 cells were cultured in the same manner as in Example 1, except that the number of cells upon producing the cell mass of the rKS56 cells were variously changed. A frequency of various cell morphological features that appeared at that time was confirmed. In FIG. 4, no lumen structure was observed in A, no assembled calices-like structure was observed and a glomerulus-like structure plus a urinary duct-like structure was observed in B, and all of the glomerulus-like structure, the urinary duct-like structure and the calices-like structure were observed in C.

As a result of the above and as shown in FIG. 5, the more sufficient differentiation-inducing property into renal tissues could be confirmed as the number of the cells used upon producing the cell mass of the rKS56 cells was increased whereas the sufficient differentiation frequency into the renal tissue is low when the number of cells is low.

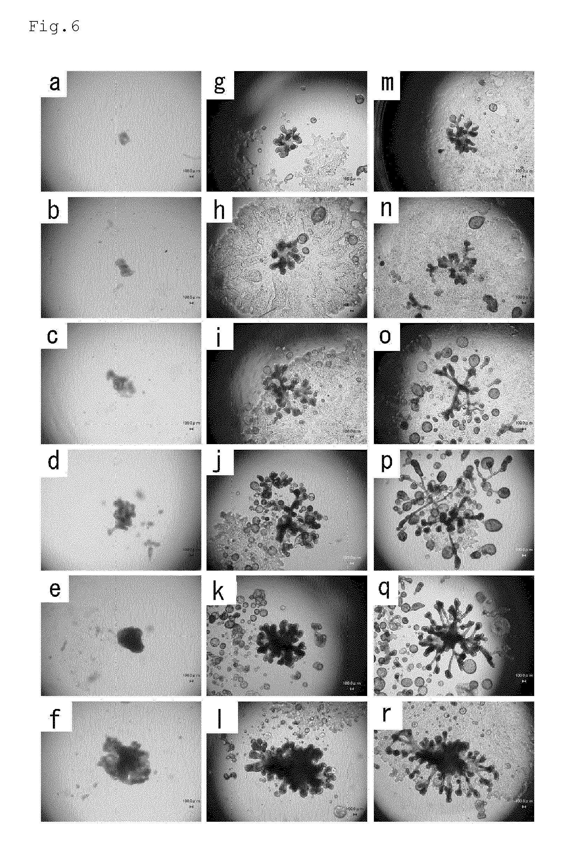

Specifically, the morphological features of the cells shown in FIG. 6 were confirmed. In FIG. 6, the number of cells upon producing the rKS56 cell mass was 6.25×10³ in a), g) and m), 1.25×10⁴ in b), h) and n), 2.5×10⁴ in c), i) and o), 5.0×10⁴ in d), j) and p), 1.0×10⁵ in e), k) and q), and 2.0×10⁵ in f), 1) and r). The morphological features on Day 1 of the culture are shown in a), b), c), d), e) and f), the morphological features on Day 7 of the culture are shown in g), h), i), j), k) and 1), and the morphological features on Day 21 of the culture are shown in m), n), o), p), q) and r).

### (Example 4) Morphological features of cultured cells when culture condition in three dimensional culture was changed

The morphological features of the various cells were confirmed when the cell mass of the rKS56 cells produced in the same manner as of the cell mass described in Example 1 was cultured in a two dimensional manner or cultured in a three dimensional manner by changing the composition of the culture medium.

The culture was carried out under the following condition a) to p). Penicillin and streptomycin (1%) were added to each culture medium. In this Example, the two dimensional culture using no scaffold was carried out when the cells were cultured in the culture medium a). The three dimensional culture was carried out when the cells were cultured in the culture medium other than a). The two dimensional culture and the three dimensional culture of the cell mass were not divided into two stages, and were carried out using the culture medium containing all the various compositions from the start of the culture.

### 1) Each culture media

a) DMEM/F12 + 10% FCS + GDNF (250 ng/mL) + b-FGF (250 ng/mL) + HGF (250 ng/mL) + EGF (500 ng/mL) + BMP7 (250 ng/mL)
b) DMEM/F12 + 10% FCS
c) DMEM/F12 + 10% FCS + GDNF (250 ng/mL)
d) DMEM/F12 + 10% FCS + b-FGF (250 ng/mL)
e) DMEM/F12 + 10% FCS + HGF (250 ng/mL)
f) DMEM/F12 + 10% FCS + GDNF (250 ng/mL) + b-FGF (250 ng/mL) + HGF (250 ng/mL)
g) DMEM/F12 + 10% FCS + EGF (500 ng/mL), change in this medium
h) DMEM/F12 + 10% FCS + BMP7 (250 ng/mL)
i) DMEM/F12 + 10% FCS + EGF (500 ng/mL) + BMP7 (250 ng/mL)
j) DMEM/F12 + 10% FCS + GDNF (250 ng/mL) + b-FGF (250 ng/mL) + HGF (250 ng/mL) + EGF (500 ng/mL) + BMP7 (250 ng/mL)
k) DMEM/F12 + 10% FCS + activin (250 ng/mL)
l) DMEM/F12 + 10% FCS + follistatin (250 ng/mL)
m) DMEM/F12 + 10% FCS + EGF (1000 ng/mL) + BMP7 (250 ng/mL)
n) DMEM/F12 + 10% FCS + HGF (250 ng/mL) + activin (250 ng/mL)
o) DMEM/F12 + 10% FCS + LIFsRα (250 ng/mL)
p) DMEM/F12 + 10% FCS + nephronectin (250 ng/mL)

### 2) Results of morphological features on Day 21 of culture

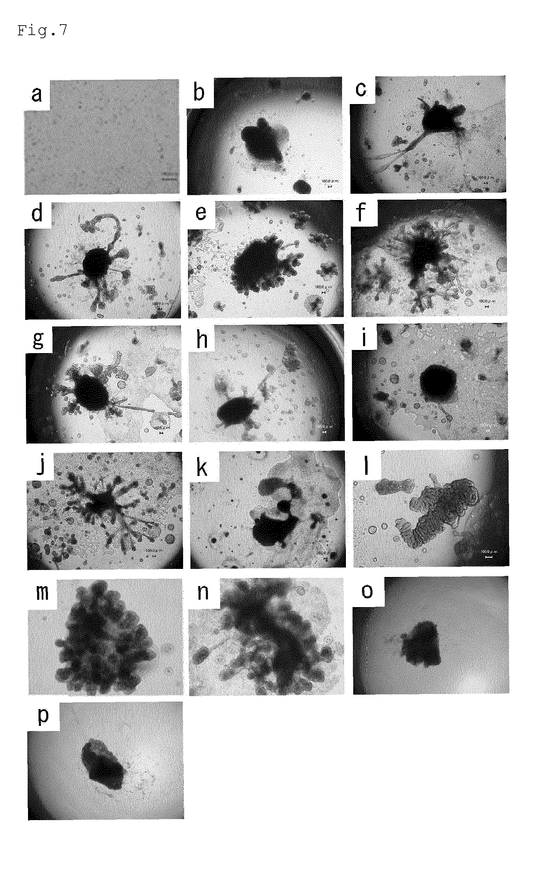

The observation result on Day 21 of the culture under various conditions are shown below (see FIG. 7). Under the condition of m), the observation result on Day 4 of the culture is shown because of logistics.

Under the culture condition of a), it is shown that morphogenesis did not occur unless the three dimensional culture was carried out (the morphological feature of the cells was changed, suggesting that the cells were differentiated).

Under the culture condition of b), it is confirmed that the morphogenesis did not occur if no growth factor was added.

Under the culture condition of c), it is confirmed that several long lumen structures were formed when GDNF alone was added.

Under the culture condition of d), it is confirmed that a thick lumen structure was formed when b-FGF alone was added.

Under the culture condition of e), it is confirmed that a lumen structure and the like was formed but its morphological feature was insufficient when HGF alone was added.

Under the culture condition of f), it is relatively confirmed that the morphogenesis occurred in the culture medium containing HGF, GDNF and b-FGF. It is also confirmed that it was unclear whether the lumen structure was thick or thin and that those closer to the normal kidney were more easily formed when the cells were cultured in the subsequent culture medium containing EGF and BMP7.

Under the culture condition of g), it is confirmed that a bended lumen structure tended to be formed when EGF alone was added.

Under the culture condition of h), it is confirmed that many glomerulus-like structures were formed on tips.

Under the culture condition of i), it is confirmed that cystic structures were formed in the mass, and a polycystic kidney-like morphological feature is confirmed.

Under the culture condition of j), amorphological feature close to a morphological feature of the kidney was confirmed.

Under the culture condition of k), it is confirmed that there was no change at all.

Under the culture condition of 1), a bended thick lumen structure was confirmed (no glomerulus-like structure).

Under the culture condition of m), it is confirmed that cystic structures were formed in the mass, and a clearer morphogenesis than the polycystic kidney-like morphological feature in i) was confirmed.

Under the culture condition of n), it is confirmed that the lumen structure could be partially formed in the culture medium containing HGF and activin.

Under the culture condition of o), it is confirmed that there was no change at all.

Under the culture condition of p), it is confirmed that there was no change at all.

As described above, it was confirmed that the degree of the morphogenesis was different depending on the condition. In particular, in i) and m), the morphological feature such as a polycystic kidney could be confirmed. The tissue cells having such a morphological feature like the polycystic kidney are useful as a pathological lesion model. Such a pathological lesion model can be practically applied as a bioassay tool in a site to be defected in vitro before testing a new drug in renal lesion, etc. , by administration in vivo, and is available as a material for screening and assay of a novel drug as a therapeutic drug, etc., for the treatment of the polycystic kidney, for example.

### (Example 5) Confirmation of morphological features on Day 4 of the three dimensional culture and confirmation of various markers

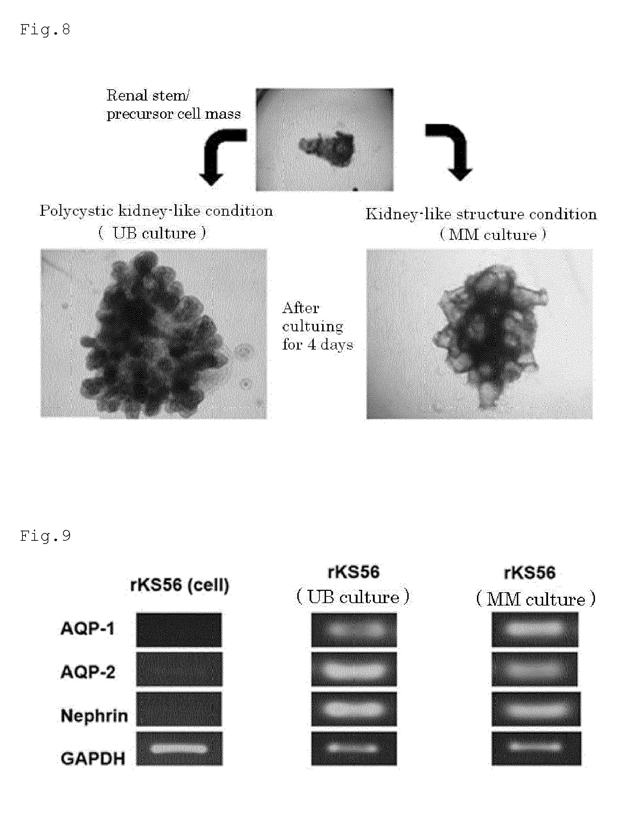

When the cells were cultured in the same manner as in Example 1, the cells were cultured under the condition for forming the polycystic kidney-like structure or the condition for forming the kidney-like structure as the culture condition in three dimensional culture. As a result, ureteric bud cells (UB) and metanephric mesenchymal cells (MM) around the UB could be observed on Day 4 of the culture (see FIG. 8). An MM condition refers to the condition for forming the kidney-like structure, and a UB condition refers to the condition for forming the polycystic kidney-like structure. Various markers on the cell surface of UB and MM were confirmed using the RT-PCR technique. Primers for performing RT-PCR for various markers are shown in Table 1.

**Table 1**

| Gene name | primer F | primer R |
|---|---|---|
| aquaporin-1 | CCTCCAGGCACAGTCTTCTC (SEQ ID NO 1) | CAGTGGCCTCCTGACTCTTC (SEQ ID NO 2) |
| Na-Ca transporter | TGGAAGCTGGTCTGTCTCCT (SEQ ID NO 3) | TGATGACATCAAGAAGGTGGTGAAG (SEQ ID NO 4) |
| nephrin | GCTCCCACCATCCGTGC (SEQ ID NO 5) | GACTATGTCCACACAACCCCCA (SEQ ID NO 6) |
| pax-2 | AGGGCATCTGCGATAATGAC (SEQ ID NO 7) | CTCGCGTTTCCTCTTCTCAC (SEQ ID NO 8) |
| gdnf | CCCGAAGATTATCCTGACCA (SEQ ID NO 9) | TAGCCCAAACCCAAGTCAGT (SEQ ID NO 10) |
| ret | GCGTCAGGGAGATGGTAAAG (SEQ ID NO 11) | CATCAGGGAAACAGTTGCAG (SEQ ID NO 12) |
| ncam | ACGTCCGGTTCATAGTCCTG (SEQ ID NO 13) | CTATGGGTTCCCCATCCTTT (SEQ ID NO 14) |
| Wt-1 | ACCCAGGCTGCAATAAGAGA (SEQ ID NO 15) | GCTGAAGGGCTTTTCACTTG (SEQ ID NO 16) |
| AQP-2 | GTGGCTGCCCAGCTGCTGGG (SEQ ID NO 17) | AGCTCCACCGACTGCCGCCG (SEQ ID NO 18) |
| bf-2 | AGGAGACAGACATCGACGTG (SEQ ID NO 19) | TGACGAAGCAGTCGTTGAG (SEQ ID NO 20) |
| gapdh | TGATGACATCAAGAAGGTGGTGAAG (SEQ ID NO 21) | TCCTTGGAGGCCATGTAGGCCAT (SEQ ID NO 22) |

Aquaporin (AQP) is a protein present on cell membranes and having fine pores, 13 types of aquaporin have been known in mammalian animals, and 6 of them are present in the kidney. Among them, AQP-1 is the marker of the proximal convoluted tubule, and AQP-2 is the marker of the distal convoluted tubule. Nephrin is a protein localized in the glomerular epithelial cells. The above markers were detected in the cells on Day 4 of the culture, suggesting that the rKS56 cells couldbe differentiated into various tissues of the kidney by the above culture (FIG. 9).

### (Example 6) Observation of cultured morphological feature

### 1) Cells cultured for two weeks

The morphological feature of a bioartificial kidney cultured in the three dimensional manner for two weeks in the same culture procedure as described in Example 1 was observed under a microscope.

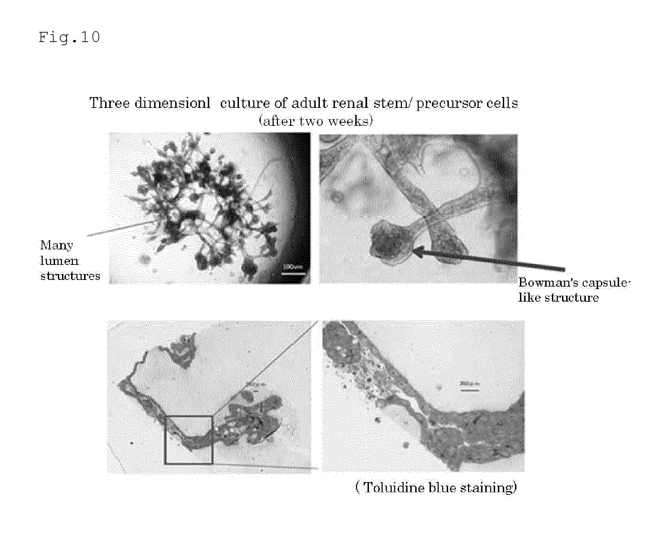

Many lumen structures like the convoluted tubule formed from the cultured cell mass were observed, and such a structure was constructed by cells having polarity. A spherical structure like the glomerulus was observed on the tip of the lumen structure (FIG. 10).

### 2) Cells cultured for three weeks

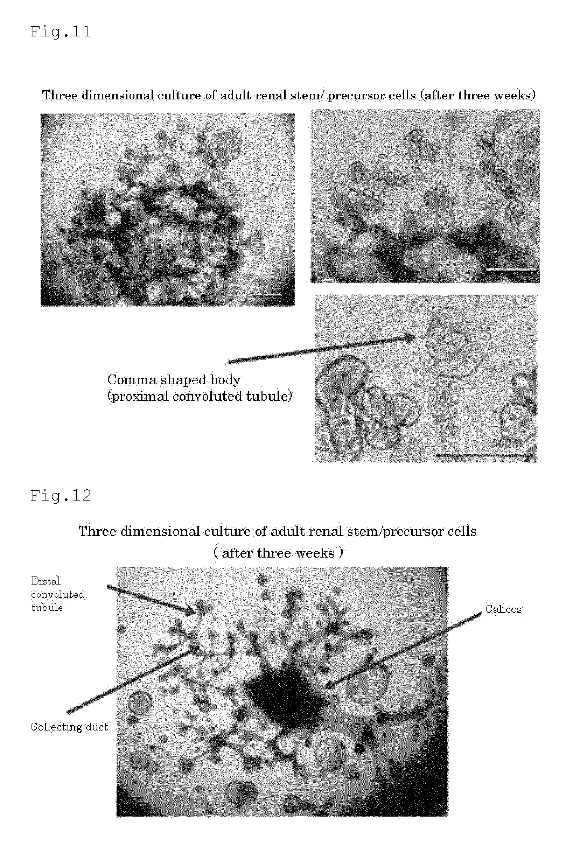

The morphological feature of a bioartificial kidney cultured in the three dimensional manner for three weeks in the above culture procedure was observed under a microscope. A change was observed in the lumen structure. The lumen structures that were similar but different in thickness and bended lumen structures, etc., were observed. The thick and bended lumen structure like the proximal convoluted tubule in the kidney, and the straight and thin lumen structures like the structures of the loop of Henle and the distal convoluted tubule were observed (FIG. 11).

### 3) Cells cultured for three weeks (observation of sphere-like structure)

The morphological feature of the bioartificial kidney cultured in the three dimensional manner for three weeks in the above culture procedure was further observed. Depending on the condition, a sphere-like structure was observed on the tip followed by the lumen structure, and these gradually assemble and gather into a calices-like structure to produce a kidney-like structure. It is considered that this structure is similar to the nephron that is the basic structural unit of the kidney and plurality of such structures assemble to exhibit the organ-like structure (FIG. 12).

### 4) Cells cultured for three weeks (observation of distal convoluted tubule structure)

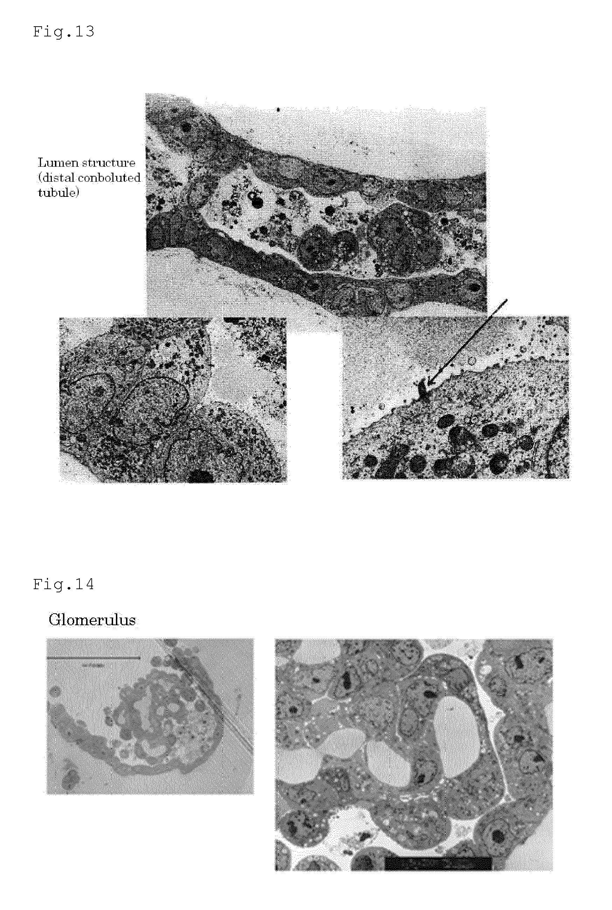

The morphological feature of the distal convoluted tubule (lumen structure) in the bioartificial kidney cultured in the three dimensional manner for three weeks in the above culture procedure was observed under an electron microscope. Lumen cells with primary cilla were observed, and it is considered that the cells were present with polarity, and the cells did not simply take the lumen structure, but had polarity and confirmed their location of one another to exist and function (FIG. 13).

### 5) Cells cultured for three weeks (observation of Bowman's capsule-like structure)

The morphological features of the glomerulus in the bioartificial kidney cultured in the three dimensional manner for three weeks in the above culture procedure was observed under an electron microscope. In the sphere-like structure at the tip of the lumen, Bowman's capsule-like structure was constructed on an outer side and the glomerulus-like structure having the lumen structure was observed therein under an electron microscope (FIG. 14).

### 6) Cells cultured for three weeks (observation of proximal convoluted tubule structure)

The morphological feature of the proximal convoluted tubule (lumen structure) in the bioartificial kidney cultured in the three dimensional manner for three weeks in the above culture procedure was observed under an electron microscope.

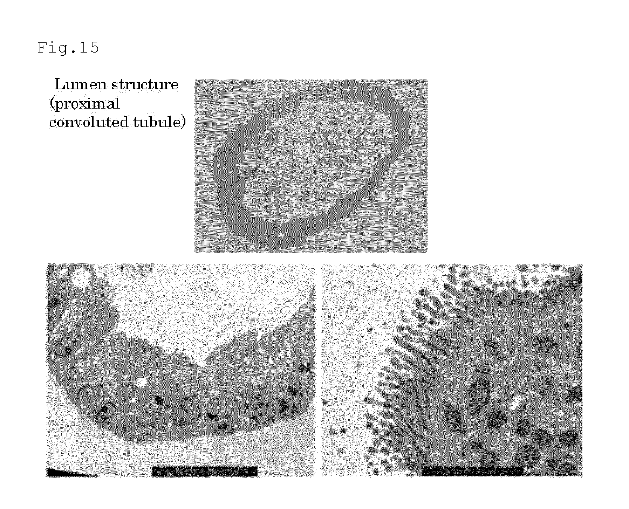

Further, the same structure as the proximal convoluted tubule structure having many villi was observed in similar lumen structures at an electron microscopic level (FIG. 15). INDUSTRIAL APPLICABILITY

As described in detail above, the differentiation of the cells can be induced by the method for inducing the differentiation of the renal stem cells into the renal tissue cells of the present invention. Specifically, the renal stem cells/precursor cells could be differentiated into the distal convoluted tubule, the glomerulus, the proximal convoluted tubule, and the like of the kidney, and the kidney-like structure could be reconstructed in vitro. This can produce the bioartificial kidney, and can further provide the material for medical purposes comprising the bioartificial organ.

Using the bioartificial kidney, it is possible to analyze what segment of a renal lesion a drug effectively acts upon or perform screening or an assay for a novel drug regarding a renal disorder therapeutic drug. By removing the renal stem cells/precursor cells from an adult, it becomes possible to produce an autologous bioartificial kidney specific to the patient himself. The bioartificial kidney and the material for medical purposes of the present invention are very useful because they can be provided for a disease to which only an organ transplant could be conventionally applied. Further, autologous bioartificial kidneys and materials for medical purposes are also excellent in safety because no rejection occurs.

## Claims

1. A method for inducing differentiation of (a) renal stem cell (s) into renal tissue cells, wherein the renal stem cell (s) is/are cultured in a three dimensional manner in a culture medium containing a hepatic cell growth factor (HGF), a glial cell line-derived neurotrophic factor (GDNF), a basic fibroblast growth factor (b-FGF), a bone morphogenetic protein 7 (BMP7) and an epithelial cell growth factor (EGF) in a base medium in the presence of a three dimensional scaffold.

2. The method for inducing the differentiation according to claim 1, wherein said culture in the three dimensional manner comprises a first step of culturing said renal stem cell(s) in a culture medium containing HGF, GDNF and b-FGF and a second step of culturing said renal stem cell (s) in a culture medium containing HGF, GDNF, b-FGF, BMP7 and EGF after 0 to 10 days have passed from the start of the culture in the first step.

3. The method for inducing the differentiation according to claim 1 or 2, wherein the cell(s) is/are cultured under a condition containing HGF, GDNF, b-FGF andBMP7 in a concentration each independently selected from 100 to 500 ng/mL and EGF in a concentration selected from 300 to 700 ng/mL in the culture medium.

4. The method for inducing the differentiation according to any one of claims 1 to 3, wherein serum is further contained in said culture medium.

5. The method for inducing the differentiation according to any one of claims 1 to 4, wherein said serum is FCS.

6. A method for inducing differentiation of (a) renal stem cell(s) into renal tissue cells comprising;
1) a step of forming a three dimensional cell mass from the renal stem cell(s); and
2) a step of culturing said cell mass in a three dimensional manner in a culture medium containing HGF, GDNF, b-FGF, BMP7 and EGF in the presence of a three dimensional scaffold.

7. The method for inducing the differentiation according to claim 6, wherein said step 2) comprises a first step of culturing said cell mass in the three dimensional manner in a culture medium containing HGF, GDNF and b-FGF in the presence of the three dimensional scaffold, and a second step of culturing the cell mass in the three dimensional manner in a culture medium containing HGF, GDNF, b-FGF, BMP7 and EGF after 0 to 10 days have passed from the start of the culture in the first step.

8. The method for inducing the differentiation according to claim 6 or 7, wherein the cells are cultured under a condition containing HGF, GDNF, b-FGF and BMP7 in a concentration each independently selected from 100 to 500 ng/mL and EGF in a concentration selected from 300 to 700 ng/mL in the culture medium.

9. The method for inducing the differentiation according to any one of claims 6 to 8, wherein serum is further contained in the culture medium in said step 2).

10. The method for inducing the differentiation according to any one of claims 6 to 9, wherein said cell mass is formed by a hanging drop method in said step 1).

11. Amethod for producing a bioartificial kidney comprising;
1) a step of forming a cell mass of renal stem cells;
2) a step of seeding the obtained cell mass on a three dimensional scaffold;
and
3) a step of culturing said cell mass in a culture medium containing HGF, GDNF, b-FGF, BMP7 and EGF on the three dimensional scaffold.

12. The method for producing the bioartificial kidney according to claim 11, wherein the cell mass is differentiated into distal convoluted tubule cells, glomerular cells and proximal convoluted tubule cells.

13. The method for producing the bioartificial kidney according to claim 11 or 12, wherein the cell mass exhibits a kidney-like structure.

14. A method for inducing differentiation of (a) renal stem cell (s) into renal tissue cells having a polycystic kidney-like morphological feature, comprising;
1) a step of forming a three dimensional cell mass from the renal stem cells; and
2) a step of culturing the obtained three dimensional cell mass of the renal stem cells in a three dimensional manner in a culture medium containing BMP7 and EGF and not containing at least one of GDNF, b-FGF or HGF in the presence of a three dimensional scaffold.

15. The method for inducing the differentiation according to claim 14, wherein the culture medium in said step 2) contains BMP7 and EGF and contains none of GDNF, b-FGF or HGF.

## Patentansprüche

1. Verfahren zum Induzieren der Differenzierung von (einer) Nierenstammzelle(n) in Nierengewebszellen, wobei die Nierenstammzelle (n) in einer dreidimensionalen Weise in einem Kulturmedium, das einen Leberzellwachstumsfaktor (HGF), einen von Gliazellen abgeleiteten neurotrophen Faktor (GDNF), einen basischen Fibroblastenwachstumsfaktor (b-FGF), ein morphogenetisches Knochenprotein 7 (BMP7) und einen Epithelzellenwachstumsfaktor (EGF) in einem Basismedium enthält, in Anwesenheit eines dreidimensionalen Gerüsts kultiviert wird/werden.

2. Verfahren zum Induzieren der Differenzierung nach Anspruch 1, wobei die genannte Kultur in dreidimensionaler Weise einen ersten Schritt des Kultivierens der genannten Nierenstammzelle(n) in einem Kulturmedium umfasst, das HGF, GDNF und b-FGF enthält, und einen zweiten Schritt des Kultivierens der genannten Nierenstammzelle(n) in einem Kulturmedium, dasHGF, GDNF, b-FGF, BMP7 und EGF enthält, nachdem 0 bis 10 Tage nach Kulturbeginn im ersten Schritt vergangen sind.

3. Verfahren zum Induzieren der Differenzierung nach Anspruch 1 oder 2, wobei die Zelle(n) unter der Bedingung kultiviert wird/werden, dass im Kulturmedium HGF, GDNF, b-FGF und BMP7 in einer Konzentration enthalten ist, die jeweils unabhängig aus 100 bis 500 ng/ml ausgewählt ist, und EGF in einer Konzentration, die aus 300 bis 700 ng/ml ausgewählt ist.

4. Verfahren zum Induzieren der Differenzierung nach einem der Ansprüche 1 bis 3, wobei im genannten Kulturmedium weiterhin Serum enthalten ist.

5. Verfahren zum Induzieren der Differenzierung nach einem der Ansprüche 1 bis 4, wobei das genannte Serum fetales Kälberserum FKS ist.

6. Verfahren zum Induzieren der Differenzierung von (einer) Nierenstammzelle(n) in Nierengewebszellen, umfassend:
1) einen Schritt des Bildens einer dreidimensionalen Zellmasse aus der/den Nierenstammzelle(n); und
2) einen Schritt des Kultivierens der genannten Zellmasse in einer dreidimensionalen Weise in einem Kulturmedium, das HGF, GDNF, b-FGF, BMP7 und EGF enthält, in Anwesenheit eines dreidimensionalen Gerüsts.

7. Verfahren zum Induzieren der Differenzierung nach Anspruch 6, wobei der genannte Schritt 2) einen ersten Schritt des Kultivierens der genannten Zellmasse in dreidimensionaler Weise in einem Kulturmedium, das HGF, GDNF und b-FGF enthält, in Anwesenheit eines dreidimensionalen Gerüsts umfasst, und einen zweiten Schritt des Kultivierens der Zellmasse in dreidimensionaler Weise in einem Kulturmedium, das HGF, GDNF, b-FGF, BMP7 und EGF enthält, nachdem 0 bis 10 Tage nach Kulturbeginn im ersten Schritt vergangen sind.

8. Verfahren zum Induzieren der Differenzierung nach Anspruch 6 oder 7, wobei die Zellen unter der Bedingung kultiviert werden, dass im Kulturmedium HGF, GDNF, b-FGF und BMP7 in einer Konzentration enthalten ist, die jeweils unabhängig aus 100 bis 500 ng/ml ausgewählt ist, und EGF in einer Konzentration, die aus 300 bis 700 ng/ml ausgewählt ist.

9. Verfahren zum Induzieren der Differenzierung nach einem der Ansprüche 6 bis 8, wobei im Kulturmedium im genannten Schritt 2) weiterhin Serum enthalten ist.

10. Verfahren zum Induzieren der Differenzierung nach einem der Ansprüche 6 bis 9, wobei die genannte Zellmasse mit einem Hängetropfenverfahren im genannten Schritt 1) gebildet wird.

11. Verfahren zur Herstellung einer bioartifiziellen Niere, umfassend:
1) einen Schritt der Bildung einer Zellmasse aus Nierenstammzellen;
2) einen Schritt des Impfens der erhaltenen Zellmasse auf ein dreidimensionales Gerüst; und
3) einen Schritt des Kultivierens der genannten Zellmasse in einem Kulturmedium, das HGF, GDNF, b-FGF, BMP7 und EGF enthält, auf dem dreidimensionalen Gerüst.

12. Verfahren zur Herstellung der bioartifiziellen Niere nach Anspruch 11, wobei die Zellmasse in distale gewundene Tubuluszellen, glomeruläre Zellen und proximale gewundene Tubuluszellen differenziert wird.

13. Verfahren zur Herstellung der bioartifiziellen Niere nach Anspruch 11 oder 12, wobei die Zellmasse eine nierenartige Struktur aufweist.

14. Verfahren zum Induzieren der Differenzierung von (einer) Nierentammzelle(n) in Nierengewebszellen mit einem polyzystischen nierenartigen morphologischen Merkmal, umfassend:
1) einen Schritt der Bildung einer dreidimensionalen Zellmasse aus den Nierenstammzellen; und
2) einen Schritt des Kultivierens der erhaltenen dreidimensionalen Zellmasse der Nierenstammzellen in dreidimensionaler Weise in einem Kulturmedium, das BMP7 und EGF enthält und nicht wenigsten eines aus GDNF, b-FGF oder HGF enthält, in Anwesenheit eines dreidimensionalen Gerüsts.

15. Verfahren zum Induzieren der Differenzierung nach Anspruch 14, wobei das Kulturmedium im genannten Schritt 2) BMP7 und EGF enthält und kein GDNF, b-FGF oder HGF enthält.

## Revendications

1. Procédé d'induction de la différentiation d'une ou de cellule (s) souche(s) rénale (s) en une ou des cellule (s) tissulaire(s) rénale(s), dans lequel la ou les cellule (s) souche(s) rénale(s) est/sont cultivée(s) d'une manière tridimensionnelle dans un milieu de culture de base contenant un facteur de croissance des hépatocytes (HGF), un facteur neurotrophique dérivé des cellules gliales (GDNF), un facteur de croissance fibroblastique basique (b-FGF), une protéine morphogénétique osseuse de type 7 (BMP7) et un facteur de croissance épithélial (EGF) en la présence d'une matrice tridimensionnelle.

2. Procédé d'induction de la différentiation selon la revendication 1, dans lequel ladite culture effectuée d'une manière tridimensionnelle comprend une première étape de culture de ladite ou desdites cellule(s) souche(s) rénale(s) dans un milieu de culture contenant HGF, GDNF et b-FGF et une deuxième étape de culture de ladite ou desdites cellule(s) souche(s) rénale(s) dans un milieu de culture contenant HGF, GDNF, b-FGF, BMP7 et EGF 0 à 10 jours après le début de la première étape de culture.

3. Procédé d'induction de la différentiation selon la revendication 1 ou 2, dans lequel la(les) cellule (s) est/sont cultivée (s) dans des conditions telles que le milieu de culture contient HGF, GDNF, b-FGF et BMP7 chacun à une concentration indépendamment sélectionnée dans une plage allant de 100 à 500 ng/ml et EGF à une concentration indépendamment sélectionnée dans une plage allant de 300 à 700 ng/ml.

4. Procédé d'induction de la différentiation selon l'une quelconque des revendications 1 à 3, dans lequel ledit milieu de culture contient également sérum.

5. Procédé d'induction de la différentiation selon l'une quelconque des revendications 1 à 4, dans lequel ledit sérum est sérum de veau foetal (SVF).

6. Procédé d'induction de la différentiation d'une ou de cellule (s) souche(s) rénale(s) en une ou des cellule(s) tissulaire(s) rénale(s) comprenant :
1) une étape de formation d'une masse cellulaire tridimensionnelle à partir de la ou des cellule(s) souche(s) rénale(s) ; et
2) une étape de culture, d'une manière tridimensionnelle, de ladite masse cellulaire dans un milieu de culture contenant HGF, GDNF, b-FGF, BMP7 et EGF en la présence d'une matrice tridimensionnelle.

7. Procédé d'induction de la différentiation selon la revendication 6, dans lequel ladite étape 2) comprend une première étape de culture, d'une manière tridimensionnelle, de ladite masse cellulaire dans un milieu de culture contenant HGF, GDNF et b-FGF en la présence d'une matrice tridimensionnelle et une deuxième étape de culture, d'une manière tridimensionnelle, de ladite masse cellulaire dans un milieu de culture contenant HGF, GDNF, b-FGF, BMP7 et EGF 0 à 10 jours après le début de la première étape de la culture.

8. Procédé d'induction de la différentiation selon la revendication 6 ou 7, dans lequel les cellules sont cultivées dans des conditions telles que le milieu de culture contient HGF, GDNF, b-FGF et BMP7 chacun à une concentration indépendamment sélectionnée dans une plage allant de 100 à 500 ng/ml et EGF à une concentration indépendamment sélectionnée dans une plage allant de 300 à 700 ng/ml.

9. Procédé d'induction de la différentiation selon l'une quelconque des revendications 6 à 8, dans lequel ledit milieu de culture contient également un sérum à ladite étape 2).

10. Procédé d'induction de la différentiation selon l'une quelconque des revendications 6 à 9, dans lequel ladite masse cellulaire est formée par une méthode des gouttelettes inversées lors de ladite étape 1).

11. Procédé de production d'un rein bioartificiel comprenant :
1) une étape de formation d'une masse cellulaire de cellules souches rénales ;
2) une étape d'ensemencement de la masse cellulaire obtenue sur une matrice tridimensionnelle ; et
3) une étape de culture de ladite masse cellulaire dans un milieu de culture contenant HGF, GDNF, b-FGF, BMP7 et EGF sur la matrice tridimensionnelle.

12. Procédé de production d'un rein bioartificiel selon la revendication 11, dans lequel la masse cellulaire est différenciée en cellules des tubes contournés distaux, cellules glomérulaires et cellules des tubes contournés proximaux.

13. Procédé de production d'un rein bioartificiel selon la revendication 11 ou 12, dans lequel la masse cellulaire présente une structure rappelant un rein.

14. Procédé d'induction de la différentiation d'une ou de cellule(s) souche(s) rénale(s) en une ou des cellule(s) tissulaire(s) rénale(s) ayant une caractéristique morphologique qui rappelle un rein polykystique, qui comprend :
1) une étape de formation d'une masse cellulaire tridimensionnelle à partir des cellules souches rénales ; et
2) une étape de culture, d'une manière tridimensionnelle, de la masse cellulaire tridimensionnelle de cellules souches rénales obtenue dans un milieu de culture contenant BMP7 et EGF et ne contenant pas un au moins facteur de type GDNF, b-FGF ou HGF en la présence d'une matrice tridimensionnelle.

15. Procédé d'induction de la différentiation selon la revendication 14, dans lequel ledit milieu de culture utilisé à ladite étape 2) contient BMP7 et EGF mais aucun facteur de type GDNF, b-FGF ou HGF.
